# EUROPEAN PATENT APPLICATION

(11) **EP 4 427 725 A1**
(43) Date of publication of application: **11.09.2024**
(21) Application number: 22887634.8
(22) Date of filing: 27.10.2022
(51) Int. Cl.: A61K 8/02, A61Q 19/00, A61Q 17/04

(54) **SKIN-ATTACHMENT PATCH AND METHOD FOR MANUFACTURING SKIN-ATTACHMENT PATCH**

(30) Priority: 01.11.2021 KR 20210148169; 19.04.2022 KR 20220048532
(71) Applicant: Biosensor Laboratories Inc., Seoul 08826 (KR)
(72) Inventor: JUNG, Min Woong, Seoul 05698 (KR); JANG, Myoung Hoon, Seoul 05501 (KR)
(74) Representative: HGF
(86) International application number: PCT/KR2022/016545
(87) International publication number: WO 2023/075439

(57) **Abstract**

Provided are a patch for skin attachment and a method of manufacturing the patch. The patch includes a base sheet, and a film disposed on the base and having a preset thickness deviation range.

## Description

### TECHNICAL FIELD

The present disclosure relates to patches to be attached to the skin and methods of using the patches.

### BACKGROUND ART

In general, a skin patch is used for skin beauty such as UV protection or moisturizing, and is attached to the skin by impregnating a general nonwoven fabric with various agents such as essence, or attached in a hydrogel state to the skin.

In addition, as a skin patch, a gel-type patch preparation using agar or starch is used. When the general nonwoven fabric is impregnated with various agents, such as essence, and attached to the skin, such as the face, the amount thereof, evaporated into air, is great, and thus, the skin patch dries completely in about 15 to 20 minutes and the adhesiveness is deteriorated, resulting in separation from the skin or deterioration of transdermal absorption of the formulation.

In addition, in the case of the hydrogel-type patch, adhesion to the skin is poor, and adhesive agents that provide adhesion often cause chemical skin troubles, and products can be produced only with limited preparations, and the manufacturing process is complicated and the aging or the like takes long, resulting in higher manufacturing costs.

In addition, in the case of a gel-type patch using agar or starch, a mesh is used as a support for the gel, and thus, the support does not perfectly support the gel, and during the user has the same on the skin, the shape is changed or broken, and after drying, the residue turned into powder, which made it difficult to maintain cleanliness.

Recently, as the outdoor leisure industry such as golf, mountain climbing, and hiking is active as well as the indoor leisure industry, there is a need to manufacture a patch that is appropriate for use during activities or even for outdoor use.

### SUMMARY

The objective of the present disclosure is to provide a patch for skin attachment, having high skin adhesion and transparency, and a method of manufacturing the same. However, the objective is an example only, and the scope of the present disclosure is not limited thereto.

Additional aspects will be set forth in part in the description which follows and, in part, will be apparent from the description, or may be learned by practice of the presented embodiments of the disclosure.

An aspect of the present disclosure provides a patch for skin attachment, the patch including a base sheet, and a film disposed on the base sheet and having a preset thickness deviation range.

In an embodiment, the film has water permeability or gas permeability, the water permeability and the gas permeability may be higher in a thinner region of the film than in a thicker region of the film.

In an embodiment, the film may include: a first layer having a first base being adjacent to the base sheet and having a first thickness, and a first recess of which thickness is smaller than the first thickness; and a second layer having a second base disposed on one side of the first layer and having a second thickness, and a second recess of which thickness is smaller than the second thickness.

In embodiment, the first recess and the second recess may at least partially overlap each other.

In an embodiment, the second recess may be placed between neighboring first recesses.

In an embodiment, at least one of the first recess and the second recess may have a mesh shape on the plane of the base sheet.

In an embodiment, the second layer may include an effective material delivered to the skin.

An aspect of the present disclosure provides a method of preparing a patch for attachment on the skin, the method including: preparing a base sheet; and forming a film by printing a raw material by screen printing on an upper surface of the base sheet, wherein, in the forming the film, printing a first layer in which the raw material is printed on a first mesh to form a first recess, and printing a second layer by printing the raw material on a second mesh, the second layer disposed on the first layer and having a second recess.

In an embodiment, in the printing the second layer, the second mesh may be disposed such that the second mesh and the first mesh overlap, or the second mesh and the first mesh are misaligned.

In addition, in the printing the second layer, the raw material including an effective material delivered to the skin may be printed.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 shows a view of a patch for skin attachment according to an embodiment of the present disclosure;
FIG. 2 is a cross-sectional view and an enlarged view taken along line II-II of FIG. 1;
FIG. 3 shows an enlarged view of portion A of a modified example of FIG. 2;
FIG. 4 illustrates operations of printing the first layer of FIG. 2;
FIG. 5 illustrates operations of printing the second layer of FIG. 2;
FIGS. 6 to 9 show enlarged views of portion C of a modified example of FIG. 5;
FIGS. 10 and 11 are views illustrating a method of manufacturing a patch for skin attachment according to an embodiment of the present disclosure; and
FIGS. 12 and 13 show graphs illustrating the ultraviolet (UV) light blocking effect of the patch for skin attachment of FIG. 1.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. The embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

The present disclosure can be subjected to various transformations and can have various embodiments. Accordingly, specific embodiments are illustrated in the drawings and described in detail in the detailed description. Effects and features of the present disclosure, and a method for achieving the same would be obvious with reference to the embodiments described below in detail in conjunction with the drawings. However, the present disclosure is not limited to the embodiments disclosed below and may be implemented in various forms.

Hereinafter, embodiments of the present disclosure will be described in detail with reference to the accompanying drawings, and when described with reference to the drawings, the same reference numerals will be applied to the same or corresponding components, and the overlapping description thereof will be omitted.

In the following embodiments, terms such as first, second, etc. are used for the purpose of distinguishing one component from another, not to limit the terms.

In the following embodiments, the singular expression includes the plural expression unless the context clearly dictates otherwise.

In the following embodiments, terms such as "include" or "have" refer to the case where the features or components described in the specification are present, and other cases where one or more other features or components can be added, are not excluded in advance.

In the following embodiments, when it is said that a part such as a film, region, or component is "on" or "above" another a part, this case includes a case where the part is directly on the other part, and a case where another film, region, component, etc. is interposed therebetween.

In the drawings, the size of the components may be exaggerated or reduced for convenience of description. For example, since the size and thickness of each component shown in the drawings are arbitrarily indicated for convenience of description, the present disclosure is not necessarily limited to the embodiments illustrated in the drawings.

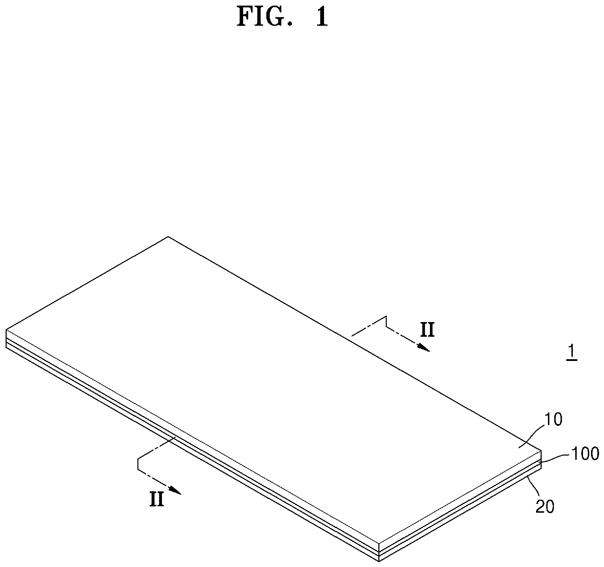

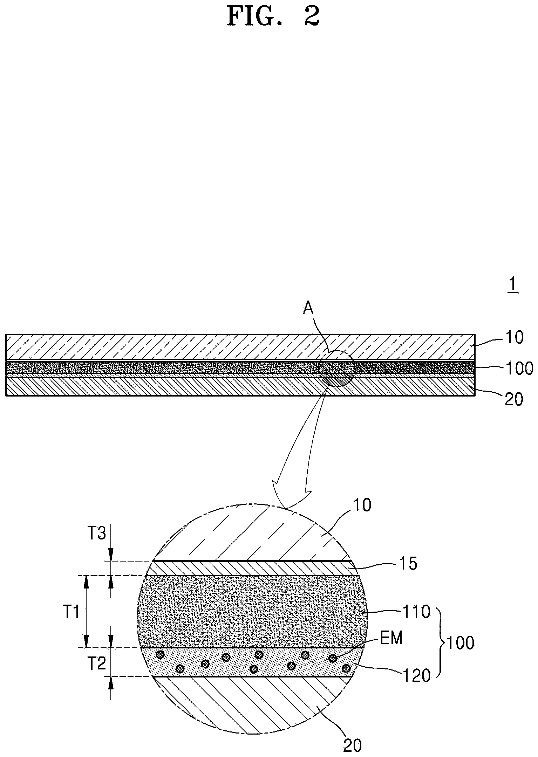

FIG. 1 shows a view of a skin attachment patch 1 according to an embodiment of the present disclosure, and FIG. 2 is a cross-sectional view and an enlarged view taken along line II-II of FIG. 1.

Referring to FIGS. 1 and 2, the skin attachment patch 1 includes a film 100. The film 100 is attached on the skin, and depending on the function of the film 100, the film 100 may deliver an effective material to the skin or may protect the skin from the external environment.

The skin attachment patch 1 may have flexibility to increase adhesion to the skin. The skin attachment patch 1 may have elasticity so that the skin attachment patch 1 may be attached to various locations on the body.

The skin attachment patch 1 may have various shapes and sizes depending on the location where the same is attached or the purpose of use. For example, the skin attachment patch 1 may have various shapes such as polygons, circles, and ovals. In an embodiment, the skin attachment patch 1 may have various sizes depending on the area the skin attachment patch 1 covers. However, hereinafter, for convenience of explanation, an embodiment in which the skin attachment patch 1 has a rectangular shape as shown in FIG. 1, will be described.

The skin attachment patch 1 may have a base sheet 10 and the film 100.

The base sheet 10 supports the film 100, one side of the base sheet 10 is exposed to the outside, and another side of the base sheet 10 may face the film 100.

In an optional embodiment, an interlayer 15 may be disposed between the base sheet 10 and the film 100. The interlayer 15 may have an adhesive force so that the film 100 is attached on the base sheet 10. The film 100 may be supported on the base sheet 10 via the interlayer 15.

The adhesive force of the interlayer 15 may be changed by an additive material. Once the film 100 is attached on the skin, the adhesive force of the interlayer 15 may be decreased to allow the base sheet 10 to be easily removed from the film 100. For example, the adhesive force of the interlayer 15 may be decreased after being in contact with a solution, and once the adhesive force of the interlayer 15 is decreased, the base sheet 10 may be easily removed from the film 100.

In an embodiment, the interlayer 15 may have decomposition properties, and may be decomposed by a liquid. For example, the interlayer 15 may be dissolved by water, and when the base sheet 10 is sufficiently wetted with water, a portion of interlayer 15 may be decomposed. When the interlayer 15 is decomposed, the adhesive force between the base sheet 10 and the film 100 may be decreased, and thus, the base sheet 10 may be easily separated from the film 100.

In another embodiment, the interlayer 15 may have a lower adhesive force than a second layer 120 of the film 100. When the base sheet 10 is removed after the second layer 120 of the film 100 is attached on the skin, since the adhesive force between the second layer 120 and the skin may be less than the adhesive force between the base sheet 10 and the interlayer 15 or the adhesive force between the first layer 110 and the interlayer 15, the base sheet 10 may be easily removed.

In an optional embodiment, the skin attachment patch 1 may further include a liner sheet 20. The liner sheet 20 may cover one side of the film 100 to protect the surface of the film 100 attached on the skin. The liner sheet 20 may be separated from the film 100 when the film 100 is attached on the skin.

The film 100 may be placed on the base sheet 10. The film 100 may be placed between the base sheet 10 and the liner sheet 20. The film 100 may have flexibility and elasticity so as to be in close contact with the skin on which the same is to be attached, and may be formed of a biocompatible material.

The film 100 may have a multi-layer structure. For example, the film 100 may include a first layer 110 and a second layer 120.

The first layer 110 may be placed adjacent to the base sheet 10. The first layer 110 may be formed of a biocompatible material.

The first layer 110 may additionally include an effective material (not shown) depending on the use of the skin attachment patch 1.

In an embodiment, the first layer 110 may include a UV blocking material. The skin attachment patch 1 may block UV rays in the area where the film 100 is attached.

In another embodiment, the first layer 110 may be transparent and may have a mark or symbol (not shown) printed therein. Depending on the transparency of the first layer 100 of the film 100 attached on the skin, the mark or symbol may be clearly seen.

The second layer 120 may be in contact with the skin and may have adhesive properties. The second layer 120 may be supported on the liner sheet 20, and may fix the first layer 110 on the skin S.

In an optional embodiment, the second layer 120 may include an effective material EM. The effective material EM may be defined as a material to be delivered into the body through the skin on which film 100 is attached, and may be, for example, drugs, cosmetics, and functional materials.

The first layer 110 may have a first thickness T1, and the second layer 120 may have a second thickness T2, which is less than that of the first layer 110.

The interlayer 15 may have a third thickness T3, the third thickness T3 may be set to be less than the second thickness T2. The interlayer 15 may be placed between the first layer 110 and the base sheet 10, and the adhesive force thereof may be less than that of the second layer 120.

The adhesive force of the interlayer 15 may be changed by an additive material. When the additive material is absorbed by the interlayer 15, the adhesive force of the interlayer 15 may be decreased. For example, when a liquid additive material is absorbed by the interlayer 15, the type of the interlayer 15 is changed to a gel or liquid, so that the base sheet 10 may slide easily on the film 100.

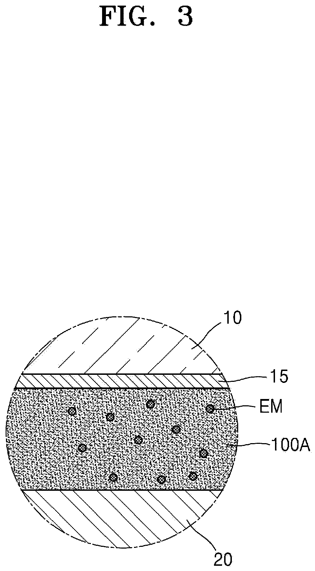

FIG. 3 shows an enlarged view of a modified example of FIG. 2, FIG. 3 shows an enlarged view of region A of FIG. 2.

Referring to FIG. 3, the patch for attachment on the skin may include a film 100A disposed between the base sheet 10 and the liner sheet 20. The interlayer 15 may be placed between the base sheet 10 and the film 100A.

The film 100A may have a single-layer structure, and a portion thereof which is adjacent to the liner sheet 20 may have an adhesive force so that the film 100A may be attached on the skin.

For example, the film 100A may be formed of polyurethane as in the first layer 110 described above.

In an optional embodiment, the film 100A may include an effective material EM. In an embodiment, the effective material EM may be, for example, drugs, cosmetics, and functional substances that can be delivered into the body through the skin S.

In an embodiment, the effective material EM may include a material that protects the skin S. In an embodiment, the effective material EM may be a material which is not delivered to the skin S, and which protects the skin from the external environment. An example of the material is a sunscreen.

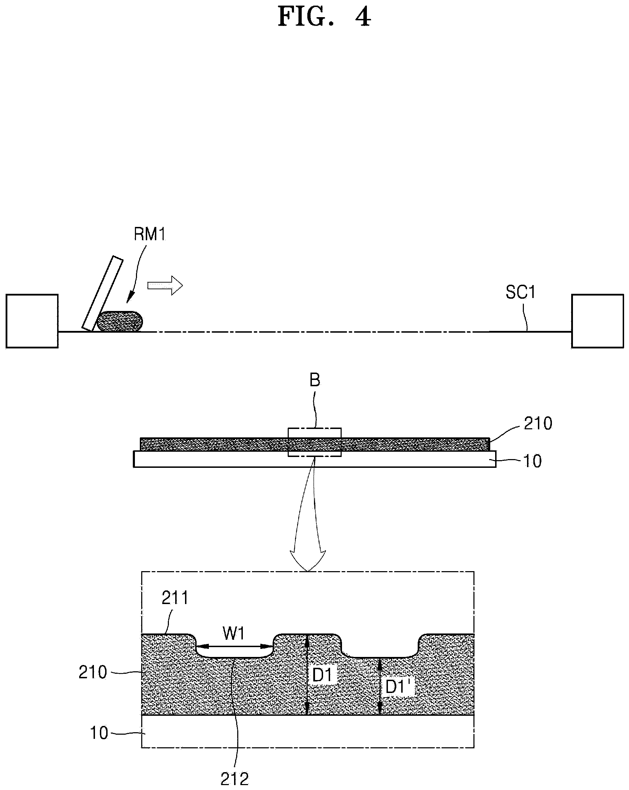

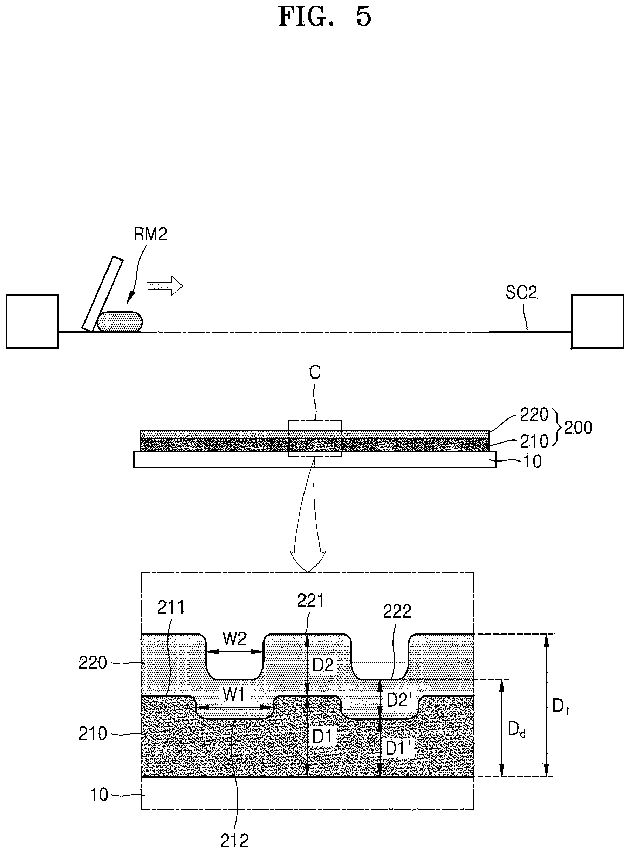

FIG. 4 illustrates operations of printing the first layer 110 of FIG. 2, and FIG. 5 illustrates operations of printing the second layer 120 of FIG. 2.

Referring to FIGS. 4 and 5, a film 200 may be formed using a screen printing method, which is well known in the art.

For example, a first layer 210 and a second layer 220, constituting the film 200, may be laminated and printed.

The first layer 210 may be formed by printing a first raw material RM1 on the base sheet 10 through a first mesh SC1. In this case, the first layer 210 may be provided to have water permeability or gas permeability.

Referring to the enlarged view of portion B of FIG. 4, the first layer 210 may be formed to have a first base 211 having a first thickness D1 and a first recess 212 having a first recess thickness D1' due to a plurality of pores and a plurality of intervals provided to a first mesh SC1.

The first layer 210 may have a thickness variation according to the width W1 and spacing of the first recess 212. According to the thickness deviation described above, portions of the first layer 210 may be provided with different degrees of water permeability or gas permeability.

Referring to FIG. 5, the second layer 220 may be disposed on the first layer 210.

The second layer 220 may be formed by printing a second raw material RM2 on the first layer 210 through a second mesh SC2. In this case, the second layer 220 may be provided to have water permeability or gas permeability.

As shown in the enlarged view of portion C of FIG. 5, the second layer 220 may be formed to include a second base 221 having a second thickness D2 and a second recess 222 having a second recess thickness D2' due to a plurality of pores and intervals thereof provided to the second mesh SC2.

Since the first layer 210 and the second layer 220 are sequentially stacked to form a single form, that is, the film 200, the second layer 220 may have a thickness deviation depending on a width W2 and spacing of the second recess 222, the width W1 and spacing of the first recess 212, and whether the second recess 222 and the first recess 212 overlap.

The film 200 has the sum of the thickness of the first layer 210 and the thickness of the second layer 220, and may have a predetermined thickness deviation range. The predetermined thickness range described above refers to a thickness range from the minimum recess thickness D_{d} to the film thickness Df.

Therefore, a portion of the film 200 corresponding to the minimum recess thickness D_{d} and a portion thereof corresponding to a thickness less than the film thickness Df, may have increased water permeability or gas permeability. That is, a portion of the film 200 having a relatively small thickness may have increased water permeability or gas permeability.

According to an embodiment, as shown in FIG. 5, the first recess 212 and the second recess 222 of the film 200 may overlap.

The first layer 210 may be printed through the first mesh SC1, thereby forming the first base 211 and the first recess 212. The second layer 220 is printed on the first layer 210 through the second mesh SC2, and the second recess 222 faces the first recess 212 and the second base 221 faces the first base 211.

The first recess 212 and the second recess 222 may each have a predetermined depth and width depending on the amount of raw material applied or the type of mesh screen.

For example, the sum of the first recess thickness D1' and the second recess thickness D2' may be the minimum recess thickness D_{d}, and the sum of the first thickness D1 and the second thickness D2 may be the film thickness Df.

Due to the overlapping of the first recess 212 and the second recess 222, a portion of the film 200 corresponding to the minimum recess thickness D_{d} may have increased water permeability or gas permeability.

In an embodiment, the recess depth of the first recess 212 may be set smaller than the recess depth of the second recess 222.

The second recess 222 and the first recess 210 overlap and thus the second recess 222 is recessed more, and the second recess thickness D2' between the second recess 222 and the first recess 212 may be decreased.

The second layer 220 may have lower water permeability or gas permeability than the first layer 210. As the second recess thickness D2' is decreased, the second layer 220 occupies less at the portion of the film 200 corresponding to the minimum recess thickness D_{d}, and the water permeability or gas permeability of the film 200 may be increased.

In an embodiment, the width W1 of the first recess 212 may be provided to be equal to or greater than the width W2 of the second recess 222.

The second recess 222 and the first recess 212 may overlap, and the width W2 of the second recess and the width W1 of the first recess 212 overlap and the width W2 is within the width W1. As the first recess 212 and the second recess 222 overlap, the degree of recess may be greater, and the layered structure of the first layer 210 and the second layer 220 may become strengthened.

Accordingly, the film 200 is formed to have a smaller minimum recess thickness D_{d}, and durability and elasticity of a portion thereof having the film thickness Df may be improved.

As the film 200 is provided to have thickness variation, durability, and elasticity, the capability of the film 200 to adhere to the skin curves may be improved, and wearability for the user may be improved.

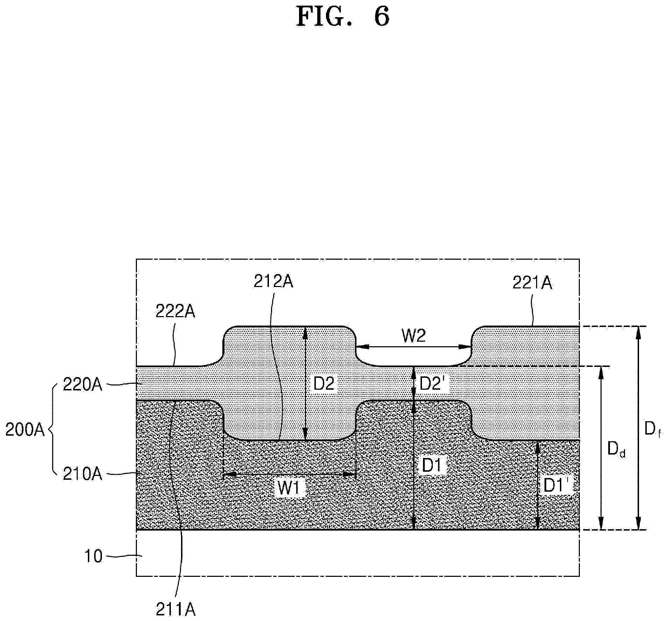

FIGS. 6 to 9 show enlarged views of portion C of a modified example of FIG. 5. The thickness deviation of the film will be described with reference to FIGS. 6 to 9.

Referring to FIG. 6, a second recess 222A of a film 200A may be disposed between neighboring first recesses 212A.

In an embodiment, in the film 200A, the second recess 222A and a first base 211A face each other, and a second base 221A and the first recess 212A face each other.

A first layer 210A is formed on the base sheet 10 by screen printing, and a second layer 220A is printed thereon, and the second recess 222A and the first recess 212A may be arranged in the zigzag order.

The first recess 212A and the second recess 222A may each have a predetermined depth and width depending on the amount of a raw material applied or the type of mesh screen.

For example, the sum of the first thickness D1 and the second recess thickness D2' is minimum recess thickness D_{d}, and the sum of the first recess thickness D1' and the second thickness D2 is the film thickness Df.

A portion of the film 200A corresponding to the minimum recess thickness D_{d}, may have increased water permeability or gas permeability.

In an embodiment, the recess depth of the first recess 212A may be set smaller than the recess depth of the second recess 222A.

Since the second recess 222A is deeply recessed and faces the first base 211A, the second recess thickness D2' can be reduced.

The second layer 220A may have lower water permeability or gas permeability than the first layer 210A. As the second recess thickness D2' is decreased, the second layer 220A occupies less at the portion of the film 200A corresponding to the minimum recess thickness D_{d}, and the water permeability or gas permeability of the film 200A may be greatly increased.

In correspondence to the low permeability of water and/or gas, the durability of the second layer 220A may be high. Accordingly, the second base 221A is located above the first recess 212A, which may improve the durability and elasticity of the film 200A. The film 200A may contribute an increase in the wearability when attached to the skin.

In an embodiment, the second layer 220A may have higher water permeability or gas permeability than the first layer 210A. In this regard, at a portion of the film 200A having the film thickness Df, the thickness D2 of the second layer 220A is greatest and the thickness D1' of the first layer 210A is smallest, so that water permeability or gas permeability thereof may be increased.

In an embodiment, the width W1 of the first recess 212A may be provided to be equal to or smaller than the width W2 of the second recess 222A.

Since the second recess 222A is supported by the first base 211A located thereunder, the layered structure of the first layer 210 and the second layer 220 may be enhanced. Accordingly, a portion of the film 200A having the film thickness Df may have increased durability and elasticity.

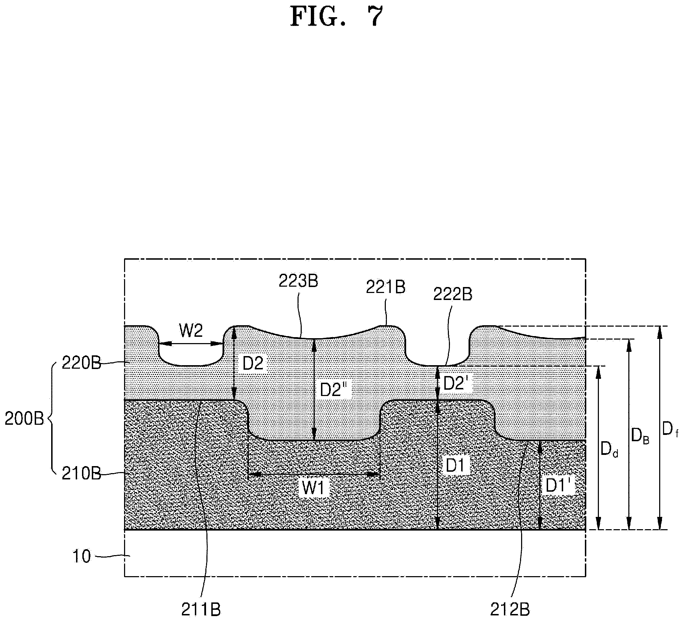

Referring to FIG. 7, as shown in FIG. 6, a second recess 222B is disposed between adjacent first recesses 212B, and the width W1 of the first recess 212B may be smaller than the width W2 of the second recess 222.

Since the width of a second base 221B is greater than the width W1 of the first recess 212B, a recess is induced by the first recess 212B in the central portion of the second base 221B, and a third recess 223B may be formed.

Since the first recess 212B and the third recess 223B overlap, a film 200B may have an induced recess thickness D_{B}.

For example, the sum of first thickness D1 and second recess thickness D2' is the minimum recess thickness D_{d}, the sum of first recess thickness D1' and a third recess thickness D2" is the induced recess thickness D_{B}, and the sum of the first thickness D1 and the second thickness D2 may be the film thickness Df.

The film 200B may be thinned in the portion having the minimum recess thickness D_{d} and the portion having the induced recess thickness D_{B}. Since the number of thin portions of the film 200B is increased, the water permeability or the gas permeability can be improved.

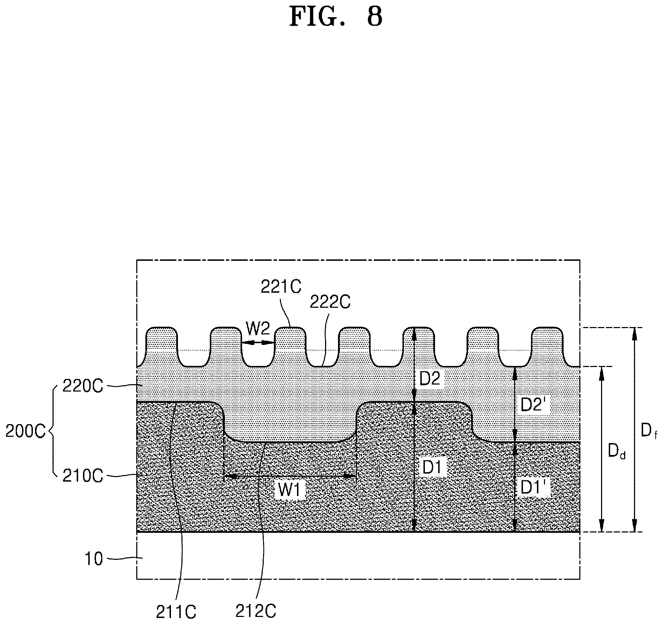

Referring to FIG. 8, the width W1 of a first recess 212C may be greater than the width W2 of a second recess 222C, and a plurality of second recesses 222C and the first recess 212C may overlap within the width W1 of the first recess 212C.

In an embodiment, the film 200C may be formed such that the spacing of the first recess 212C of a first layer 210C may be greater than the spacing of the second recess 222C of a second layer 220C.

The first layer 210C is printed on the base sheet 10 through the first mesh SC1, and a first base 211C and the first recess 212C are formed. The second layer 220C is printed on the first layer 210C through the second mesh SC2, and the mesh interval of the second mesh SC2 may be narrower than the mesh interval of the first mesh SC1.

A portion of the film 200C corresponding to where the first recess 212C and the second recess 222C overlap, has a minimum recess thickness D_{d}, and a portion thereof corresponding to where the first base 211C and a second base 221C overlap, has a maximum film thickness D_{f}.

For example, the sum of the first recess thickness D1' and the second recess thickness D2' may be the minimum recess thickness D_{d}, and the sum of the first thickness D1 and the second thickness D2 may be the film thickness Df.

The film 200C includes as many portions that have smaller thicknesses than the film thickness Df as the number of the second recess portions 222C, so that water permeability or gas permeability thereof can be improved.

In an embodiment, the second layer 220C may have lower water permeability or gas permeability than the first layer 210C. In particular, portions of the film 200C corresponding to where a plurality of second recesses 222C face the first base 211C, may have improved water permeability or gas permeability.

In correspondence to the low permeability of water and/or gas, the durability of the second layer 220C may be high. Due to the plurality of second bases 221C in a dense arrangement, the film 200C may have improved durability, elasticity and wearability.

In one or more embodiments, the second layer 220C may have higher water permeability or gas permeability than the first layer 210C. In particular, portions of the film 200C corresponding to where a plurality of second recesses 222C and the first recess 212C overlap, may have improved water permeability or gas permeability.

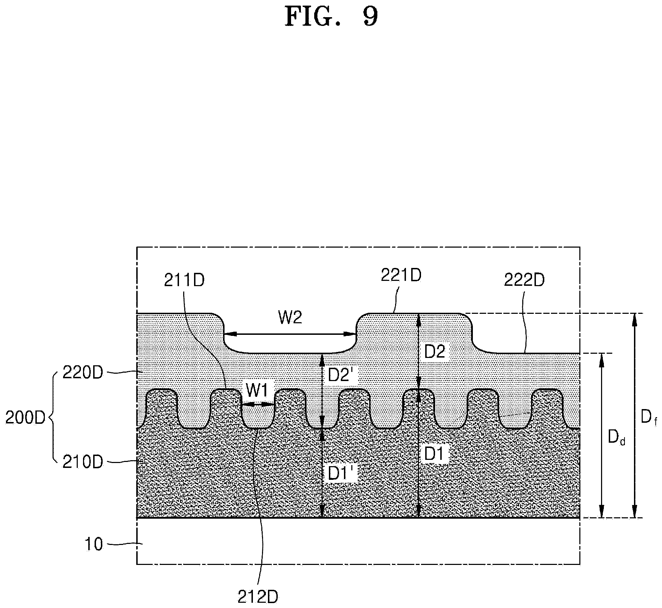

Referring to FIG. 9, the width W1 of a first recess 212D may be smaller than the width W2 of a second recess 222D, and a plurality of first recesses 212D and the second recess 222D may overlap within the width W2 of the second recess 222D.

In an embodiment, a film 200D may be formed such that the spacing of the first recess 212D of a first layer 210D may be narrower than the spacing of the second recess 222D of a second layer 220D.

The first layer 210D is printed on the base sheet 10 through the first mesh SC1, and a first base 211D and the first recess 212D are formed. The second layer 220D is printed on the first layer 210D through the second mesh SC2, and the mesh interval of the second mesh SC2 may be greater than the mesh interval of the first mesh SC1.

A portion of the film 200D corresponding to where the first recess 212D and the second recess 222D overlap, has a minimum recess thickness D_{d}, and a portion thereof corresponding to where the first base 211D and a second base 221D overlap, has a maximum film thickness D_{f}.

For example, the sum of the first recess thickness D1' and the second recess thickness D2' may be the minimum recess thickness D_{d}, and the sum of the first thickness D1 and the second thickness D2 may be the film thickness Df.

A portion of the film 200D where the second recess 222D and the first recess 212D overlap, is included within the second recess 222D, and a portion of the film 200D of which thickness is small due to the second recess 222D, may have improved water permeability and gas permeability.

In an embodiment, the second layer 220D may have lower water permeability or gas permeability than the first layer 210D. In this regard, since a portion of the first layer 210D having the first thickness D1' may have high water and/or gas permeability, portions of the film 200D corresponding to where the first recess 212D and the second recess 222D overlap, may have improved water permeability or gas permeability.

In correspondence to the low permeability of water and/or gas, the durability of the second layer 220D may be high. Since the first layer 210D includes a plurality of first recesses 212D, the durability thereof may be reduced. However, since the first layer 210D is supported by the second layer 220D, the layered structure may be strengthened. Accordingly, durability, elasticity, and wearability of the film 200D may be improved.

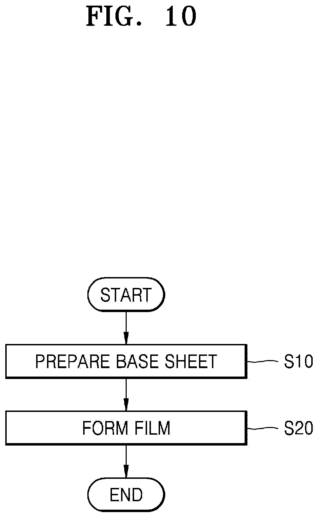

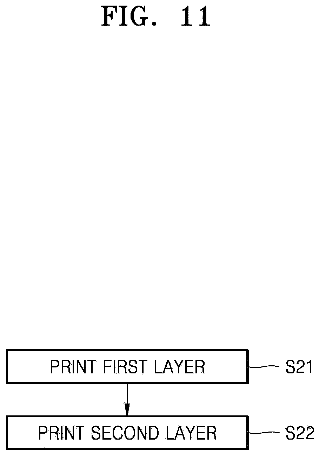

FIGS. 10 and 11 are views illustrating a method of manufacturing a patch for skin attachment according to an embodiment of the present disclosure; and

Referring to FIGS. 4, 5, 10, and 11, the method of manufacturing the skin attachment patch 1 may include preparing a base sheet (S10) and forming a film (S20).

In the preparing the base sheet (S10), the base sheet 10 may be provided such that while being suitable for screen printing of the film 200, peeling between the base sheet 10 and the film 200 occurs easily.

The forming the film (S20) may include printing the first layer (S21) and printing the second layer (S22).

Referring to FIG. 5, in the printing the first layer (S21), the first raw material RM1 is printed on the first mesh SC1 to form the first layer 210, and the first layer 210 may have a thickness range between the first thickness D1 and the first recess thickness D1'.

In the printing the second layer (S22), a second raw material RM2 may be printed on a second mesh SC2 to form the second layer 220 on an upper surface of the first layer 210.

In the printing the second layer ( S22 ), the second mesh SC2 may be disposed such that the second mesh SC2 and the first mesh SC1 overlap, or the second mesh SC2 and the first mesh SC1 are misaligned.

Accordingly, the second layer 220 may be formed to have the second thickness D2 and the second recess thickness D2' according to the arrangement and width of the second recess 222 and the first recess 212.

In the printing the second layer (S22), the second raw material RM2 containing the effective material delivered to the skin may be printed. the effective material is defined as a substance delivered into the body through the skin, and may be, for example, drugs, cosmetics, and functional substances. Accordingly, when the user adheres the film 200 to the skin, the skin receives the effective material from the second layer 220, and may exhibit skin care and health support effects.

Since the film 200 is used for bonding to the skin, the film 200 may be provided to allow moisture, such as sweat generated from the skin, to be discharged and evaporated to the outside of the film 200. Therefore, a performance test for water permeability is required.

The water permeability test of the film 200 was performed according to the water-film contact method. In the water-film contact method, the maximum amount of water is housed in the experimental vessel so as to make almost no air layer formed between the film 200 disposed on the upper part of the vessel and the water. Accordingly, the air resistance to the film 200 may be removed and water permeation performance may be tested.

As a result of performing the water permeability test by the method under the temperature condition of 32.2 °C, the water permeability of the film 200 was measured to be 7460 g/m²*day. Comparing this result with the actual amount of perspiration, the performance of the film 200 with respect to sweat discharge and evaporation can be confirmed.

In general, during resting in a cool environment, the amount of perspiration is 270 g/m²*day, during extreme exercise, the amount of perspiration is 2880 g/m²*day (based on 20°C), and during exercising in a relatively hot environment, the amount of perspiration is 5500 g/m² *day. Since the water permeability of the film 200 according to an embodiment of the present disclosure is 7460 g/m²*day, it was confirmed that most of the sweat generated from the skin can be discharged to the outside of the film 200 and evaporated.

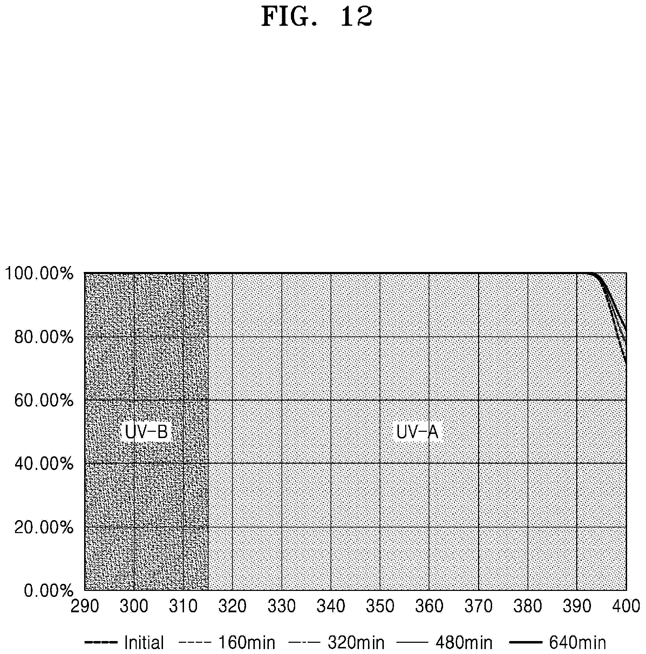

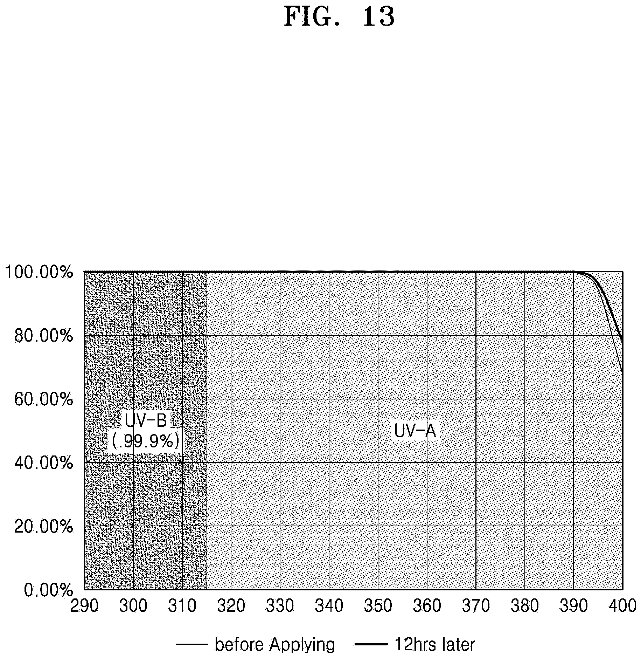

FIGS. 12 and 13 show graph illustrating the ultraviolet (UV) light blocking effect of the patch for skin attachment of FIG. 1.

The skin attachment patch of FIGS. 12 and 13 includes a UV blocking material included in a film, and may have UV blocking performance, water resistance (FIG. 12) and blocking retention (FIG. 13). In the graphs, the x-axis represents the wavelength, and the y-axis is defined as the blocking rate.

When the thickness of the skin attachment film was 25 µm, the UV protection factor (UPF) of the film was 343, the UV-B blocking ratio was 99.9% and the UV-A blocking ratio was 95.8%.

When the thickness of the skin attachment film was 35 µm, the UV protection factor (UPF) of the film was 343, the UV-B blocking ratio was 99.9% and the UV-A blocking ratio was 97.4%.

In the water resistance test, the skin attachment patch was fixed on a 96 well plate, and an experimental environment was provided such that both the inner and outer surfaces of the skin attachment film were immersed in water. Immersing (20 min) and drying (20 min) were repeatedly performed.

In the graph of FIG. 12, 160 min refers to a case where immersing and drying were performed 8 times, 320 min refers to a case where immersing and drying were performed 16 times, 480 min refers to a case where immersing and drying were performed 24 times, and 640 min refers to a case where immersing and drying were performed 32 times. Even when the patch for attachment on the skin was repeatedly immersed and dried, the UV protection efficiency was not decreased. In particular, it was confirmed that even if the patch for attachment on the skin was repeatedly immersed and dried, almost 100% blocking efficiency was maintained in the UV-B wavelength band, and the UV-A wavelength band of 390 nm or lower.

In an embodiment, even when physical stimulation with ultrasound is applied to the skin attachment patch, the UV blocking efficiency is not decreased.

In the blocking retention test, a skin attachment patch was attached on the skin, and the UV blocking effect was measured at the initial time point and after 12 hours. Even after 12 hours have elapsed since the skin attachment patch is attached on the skin, the UV protection efficiency is not decreased.

UV protection products that are applied to the skin need to be applied continuously at 2 hour intervals to maintain UV protection performance. This is because, even when a cream-type sunscreen product is applied, the blocking rate is decreased due to sweat, physical contact, a decrease in the effectiveness of the sunscreen, and the absorption of the sunscreen into the skin.

In the case of the skin-attached patch of the present disclosure, UV blocking power can be maintained while the patch is attached to the skin. Unlike cream-type sunscreens of the prior art, in the case of the skin attachment patch of the present disclosure, UV protection efficiency may not be decreased by external environments such as water after being attached once, and UV rays may be continuously blocked.

Table 1 shows an experimental result confirming whether the following test items were detected after exposing the skin attachment patch of the present disclosure to water for 24 hours.

The skin attachment patch according to the present disclosure may be selected from ingredients having UV absorption/blocking functions. In this test, a plurality of skin attachment patches including at least one of the ingredients in Table 1 were used, and in each test, the following components were not detected even when exposed to water for a long time.

**[Table 1]**

| Test Items | Unit | Result | R.L | Remarks |
|---|---|---|---|---|
| Ethylhexylmethoxycinnamate | µg/g | nondetection | 10 | In House Method (HPLC-UVD) |
| Butylmethoxydibenzoylmethane | µg/g | nondetection | 10 | |
| homosalate | µg/g | nondetection | 10 | |
| Ethylhexylsalicylate | µg/g | nondetection | 10 | |
| Phenylbenzimidazole sulfonic acid | µg/g | nondetection | 10 | |
| Octocrylene | µg/g | nondetection | 10 | |
| Benzophenone-4 | µg/g | nondetection | 10 | |
| Benzophenone-8 | µg/g | nondetection | 10 | |

When the skin attachment patch of the present disclosure is exposed to water for a long time, a corresponding component is not detected. It was confirmed that the component was contained inside the film and did not leak to the outside.

The skin attachment patch according to the present disclosure may cause less skin troubles because the ingredients thereof do not leak even when exposed to the external environment.

As such, the present disclosure has been described with reference to the embodiments shown in the drawings, but the embodiments are only an example, and those of ordinary skill in the art would understand that various modifications and equivalent other embodiments are possible therefrom. Therefore, the technical protection scope of the present disclosure should be determined by the technical idea of the appended claims.

According to the patch for skin attachment and a method of manufacturing the same according to an embodiment of the present disclosure, the film of the patch for skin attachment may be manufactured to have a thickness variation, so that the permeability of moisture and gas in a thinner region may be improved. Accordingly, the feeling of use may be improved, and the comfort when wearing the same on the skin may be increased.

The skin attachment patch and the method of manufacturing the same according to an embodiment of the present disclosure may have improved functionality. The part thereof in contact with the skin contains effective materials that are delivered to the skin, so that skin troubles may not occur and the cosmetic effect may be enhanced. A film containing a UV blocking material may have high waterproof performance, high waterproof retention performance, and high UV blocking performance.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope as defined by the following claims.

## Claims

1. A patch for attachment on the skin, comprising:
a base sheet; and
a film disposed on the base sheet and having a preset thickness deviation range.

2. The patch of claim 1, wherein
the film has water permeability or gas permeability, and
a portion of the film having a smaller thickness has higher water permeability or gas permeability than a portion thereof having a greater thickness.

3. The patch of claim 1, wherein the film includes:
a first layer adjacent to the base sheet and having a first base having a first thickness and a first recess of which thickness is smaller than the first thickness; and
a second layer disposed on one side of the first layer, and having a second base having a second thickness and a second recess of which thickness is smaller than the second thickness.

4. The patch of claim 3, wherein the first recess and the second recess at least partially overlap each other.

5. The patch of claim 3, wherein the second recess is disposed between neighboring first recesses.

6. The patch of claim 3, wherein at least one of the first recess and the second recess has a mesh shape on the plane of the base sheet.

7. The patch of claim 3, wherein the second layer contains an effective material to be delivered to the skin.

8. A method of preparing a patch for attachment on the skin, the method comprising:
preparing a base sheet; and
forming a film by printing a raw material by screen printing on an upper surface of the base sheet, wherein
in the forming the film,
printing a first layer in which the raw material is printed on a first mesh to form a first recess; and
printing a second layer by printing the raw material on a second mesh, the second layer disposed on the first layer and having a second recess.

9. The method of claim 8, wherein, in the printing the second layer, the second mesh is disposed such that the second mesh and the first mesh overlap, or the second mesh and the first mesh are misaligned.

10. The method of claim 8, wherein in the printing the second layer, the raw material including an effective material to be delivered to the skin, is printed.
